Europäisches Patentamt

European Patent Office

Office européen des brevets

(11) Publication number: **0 395 817**
**A1**

## (12) EUROPEAN PATENT APPLICATION

(21) Application number: 89312220.0

(51) Int. Cl.5: **A61M 5/00**

(22) Date of filing: 24.11.89

(30) Priority: 02.05.89 CA 598416

(43) Date of publication of application:
07.11.90 Bulletin 90/45

(84) Designated Contracting States:
AT BE CH DE ES FR GB GR IT LI LU NL SE

(71) Applicant: ECO CORPORATION
6725 Airport Road Suite 502
Mississauga Ontario L4V 1V2(CA)

(72) Inventor: Warrington, John E.
36 Alder Road
Toronto Ontario(CA)

(74) Representative: Carter, Gerald
Arthur R. Davies & Co. 27 Imperial Square
Cheltenham, Gloucestershire GL50 1RQ(GB)

(54) Injection needle protector.

(57) A protector for an injection needle is provided, to safeguard the operator against accidental contact or puncturing of the hand when storing a needle away before or after use. The protector has a central tube (34) in which a conventional, narrow needle (18) covering sleeve (22) is received as an interference fit and a radially extending skirt (36) of frusto-conical or similar shape, surrounding the central tube (34) and serving to guide the incident needle (18) towards and into the covering sleeve (22) even in cases of gross misalignment of the incident needle (18).

EP 0 395 817 A1

This invention relates to medical injection apparatus, and more particularly to a novel form of protector for an injection needle.

Injection needles are normally detachable from injection syringes so that the syringe can be used repeatedly whereas the needle is only used once or twice. As soon as an injection needle has been used, it is highly desirable that it should be appropriately protected. Protection of the needle is of course desirable at all times, but it is particularly important after use when the needle may be contaminated with infectious microorganisms.

It is common to provide a narrow protective sleeve for an injection needle, into which the needle can be inserted immediately after use, before or after detachment from the syringe. Then the needle may be discarded while protected by its sleeve. An operator is however at serious risk in using such a sleeve. When holding the sleeve in one hand and the needle or syringe in the other, the chances of puncturing the fingers holding the sleeve, due to misalignment of the approach of the needle, are significant. With a contaminated needle, e.g. carrying infectious body fluids, the consequences can be serious.

It is an object of the present invention to provide a novel and effective protection means for an injection needle.

Accordingly, the present invention provides an injection needle protector which has an inner, needle receiving sleeve into which the needle is received and the shank of the needle is sealed and an outer shield having a central tube into which the sleeve is an interference fit, and a skirt surrounding the tube, acting as a guide for the needle to enter the inner sleeve. The skirt normally is frusto-conical, with a hard smooth inner surface along which the needle point will slide without significant damage, within the tube at its centre.

Thus according to one aspect of the present invention, there is provided protection means for attachment to an injection needle of the type having an injection needle shank and a holding stem for the needle, said protection means comprising:
an inner sleeve having an open proximal end sized to provide an interference fit over the holding stem for the needle, an elongated body within which to receive the needle shank, and a closed distal end;
an outer shield having a central tube and a skirt extending radially outwardly and proximally from the proximal end of the central tube;
the proximal end of the inner sleeve being adapted to fit within the proximal end of the tube substantially in axial alignment therewith.

According to a further aspect of the invention, there is provided injection needle protection means adapted to receive an injection needle, said protection means comprising a frusto-conical skirt having a continuous, smooth inner surface of revolution converging in a direction away from the approach of a needle to be received therein, said surface being adapted to cause sliding movement of an incident needle point therealong; and an integral central tube at the convergence of the frusto-conical skirt and extending away from said skirt, said central tube being dimensioned to receive a needle protection sleeve in interference fit therewith.

Preferably the inner sleeve and outer shield are separable from one another, so that the inner sleeve, in which the needle is effectively sealed, and the needle can be discarded safely as a unit. Preferably also, the central tube and skirt of the outer shield are of integral one-piece construction for ease and economy of manufacture.

A specific embodiment of the invention is shown, by way of non-limiting example, in the accompanying drawings, in which:

FIGURE 1 is an exploded perspective view of an injection syringe assembly incorporating a specific preferred embodiment of the present invention;

FIGURE 2 is a perspective view of the protection means of FIGURE 1.

In the drawings, like reference numerals indicate like parts.

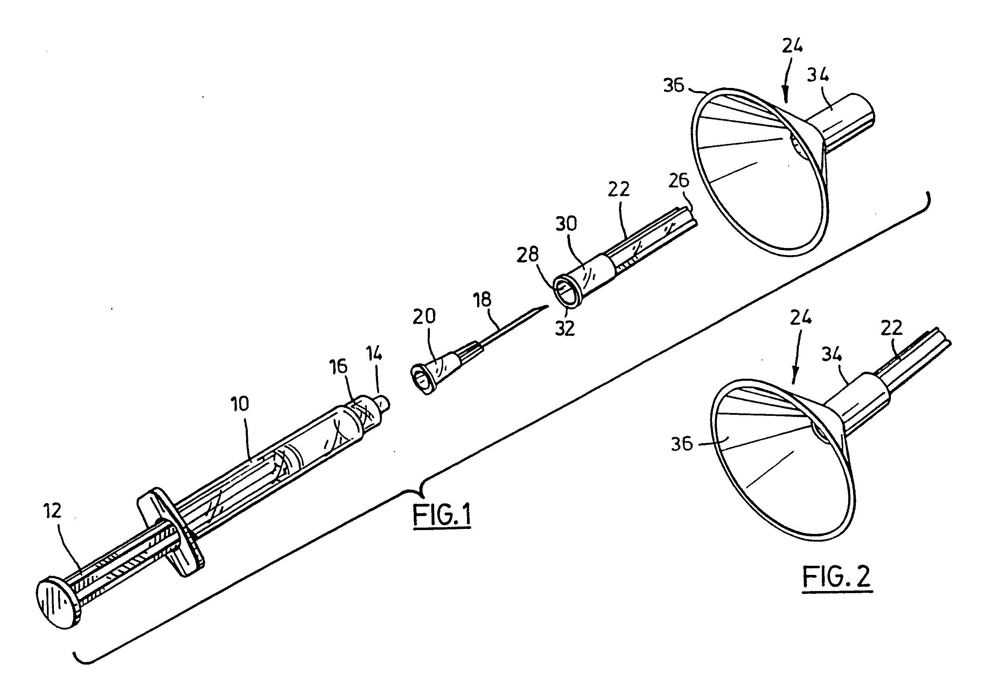

FIGURE 1 shows a conventional injection syringe having a reservoir 10 containing a reciprocable plunger 12 to expel injectable liquid therefrom. The outlet end of the reservoir 10 has an outlet nozzle 14 and a holding stem 16. An injection needle 18 with enlarged integral inlet 20 is releasably fitted onto holding stem 16 by interference fit of the inlet 20 over the outlet nozzle 14. Then the injection syringe is ready for use. The needle 18 and its integral inlet 20 are disposable items, intended for single use and then discard, or at least sterilization before re-use for a limited number of times.

Before and after use, the syringe and attached needle, or the needle itself, needs to be stored in a safe, protected condition. Accordingly, in the illustrated embodiment there is provided an inner sleeve 22 to receive and enclose the needle 18, and an outer shield 24. The sleeve 22 has a closed distal end 26 and an open proximal end 28. The sleeve 22 has an enlarged portion 30 at its proximal end 28 which provides a close, sealing interference fit around the inlet 20 of the needle 18. It also has a protruding end rim 32 on its proximal end. With the inlet 20 of the needle and the proximal end 28 of the sleeve 22 engaged, the needle 18 is sealed within the sleeve 22.

The outer shield 24 has a central tube 34 and a frusto-conical skirt 36, so that it has the general shape and appearance of a funnel, with the central tube 34 protruding away from the syringe. The tube 34 is dimensioned to fit closely over the enlarged end portion 30 of sleeve 22, but to abut against rim 32 thereof.

In use, as illustrated in FIGURE 2, the sleeve 22 is assembled with the outer shield 24, by pushing the sleeve 22 down the tube 34 from the proximal end thereof, until sealing interference fit of enlarged portion 30 within the sleeve 22, and abutment of the rim 32 against the end of sleeve 22 is achieved. The proximal end of the tube 34 is then substantially in axial alignment with the proximal end 28 of the sleeve. Most of sleeve 22 then protrudes beyond the distal end of the tube 34. Now the operator can readily insert the needle into its protective sleeve 22. The conical skirt 36 safeguards against any but the wildest misalignments of the needle and the proximal end 28 of the sleeve 22, and serves to guide the needle into proper entrance to the sleeve. The operator's fingers holding the outer shield 24 are thus well protected from contact and accidental puncturing with the needle 18 as it is sheathed. The use of protective sleeve 22 or the like alone offers no such assurance of protection against accidental contact and puncturing and careless use of the needle.

Conveniently, the parts are made from readily available sterilizable thermoplastic such as food grade polyethylene or nylon. Their simplicity of shape and design means that they are readily and economically made by standard molding techniques, from relatively cheap starting materials. The interior conical surface of the skirt 36 is arranged to be sufficiently hard and smooth so that a needle point contacting it will slide readily along it to the centre of the tube, without puncturing the skirt surface or damaging the needle to any significant extent. Because of the axial alignment of the sleeve and tube proximal ends, the needle tip is guided to enter the sleeve 22.

Whilst it is preferred to have a separable inner sleeve and outer shield as illustrated, they can if desired be combined as a single integral unit. Since inner sleeves tend to be standard items conventionally supplied along with injection needles for protection thereof, it is in most cases more convenient to prepare and supply the outer shield as a separate item. Moreover, since the outer shield has little if any contact with the needle itself, and is of a shape and size to be readily sterilizable, it can be conveniently re-used to a greater extent than the inner sleeve.

## Claims

1. Protection means for attachment to an injection needle of the type having an injection needle shank and a holding stem for the needle shank, said protection means comprising:

an inner sleeve having an open proximal end sized to provide an interference fit over the holding stem of the needle, an elongated body within which to receive the needle shank, and a closed distal end;

an outer shield having a central tube and a skirt extending radially outwardly and proximally from the proximal end of the central tube;

the proximal end of the inner sleeve being adapted to fit within the proximal end of the tube substantially in axial alignment therewith.

2. Protection means according to claim 1 wherein the radially outwardly extending skirt is frusto-conical.

3. Protection means according to claim 2 wherein the central tube and skirt of the outer shield are of integral one-piece construction.

4. Protection means according to claim 3 wherein the inner sleeve fits within the tube as a tight interference fit with effective sealing, the inner sleeve and the tube being separable from one another.

5. Protection means according to claim 3 wherein the inner sleeve and the outer shield are of integral, one-piece construction.

6. An injection syringe and protection means therefor, comprising:

a reservoir having an inlet end and an outlet end and containing a reciprocal plunger for expelling liquid from the reservoir;

a needle having a holding stem and a needle shank, releasably secured to the outlet end of the reservoir;

protection means comprising an inner sleeve enclosing the injection needle, and an outer shield having a central tube engaging said inner sleeve and a frusto-conical skirt tapering in diameter towards said central tube and extending towards the reservoir;

the protection means being removably attached to the needle holding stem.

7. An injection needle protection means adapted to receive an injection needle, said protection means comprising:

a frusto-conical skirt having a continuous, smooth inner surface of revolution converging in a direction away from the approach of a needle to be received therein, said surface being adapted to cause sliding movement of an incident needle point therealong;

and an integral central tube at the convergence of the frusto-conical skirt and extending away from said skirt, said central tube being dimensioned to receive a needle protection sleeve in interference fit therewith.

FIG.1

FIG.2

European Patent Office

# EUROPEAN SEARCH REPORT

Application Number

EP 89 31 2220

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 485 918 (MAYER) <br> * Figures 1-3; column 3, lines 4-32; column 4, lines 2-5 * | 1-3 | A 61 M 5/00 |
| Y | | 7 | |
| A | | 4 | |
| | --- | | |
| X | US-A-4 781 697 (SLAUGHTER) <br> * Figures 5,6; column 4, lines 10-14 * | 1,3,4 | |
| Y | | 7 | |
| | --- | | |
| A | EP-A-0 284 183 (SANDHAUS) <br> * Figure 11; column 8, lines 27-33 * | 5,6 | |
| | --- | | |
| A | US-A-4 737 149 (GILLILAN) <br> * Figures 1,6 * | 1 | |
| | --- | | |
| A | DE-A-3 433 359 (KREISZ) <br> * Whole document * | 1 | |
| | --- | | |
| A | GB-A-2 186 800 (SELDOREN LTD) <br> * Figure 2 * | 1 | |
| | ----- | | **TECHNICAL FIELDS SEARCHED (Int. Cl.5)** <br><br> A 61 M |

The present search report has been drawn up for all claims

| Place of search | Date of completion of the search | Examiner |
|---|---|---|
| THE HAGUE | 10-08-1990 | SEDY, R. |